# EUROPÄISCHE PATENTSCHRIFT

(11) **EP 2 274 111 B1**
(45) Veröffentlichungstag und Bekanntmachung des Hinweises auf die Patenterteilung: **24.07.2013**
(21) Anmeldenummer: 09728866.6
(22) Anmeldetag: 18.03.2009
(51) Int. Cl.: B08B 9/30, A23L 3/04, A23L 3/3445, A23L 3/3589, A61L 2/20, A61L 2/24, A61L 2/18, A61L 2/22

(54) **VORRICHTUNG ZUM BEHANDELN VON FLASCHEN ODER DERGLEICHEN BEHÄLTERN**
DEVICE FOR TREATING BOTTLES OR SIMILAR CONTAINERS
DISPOSITIF SERVANT À TRAITER DES BOUTEILLES OU RÉCIPIENTS ANALOGUES

(30) Priorität: 04.04.2008 DE 102008017524
(43) Veröffentlichungstag der Anmeldung: 19.01.2011
(73) Patentinhaber: KHS GmbH, 44143 Dortmund (DE)
(72) Erfinder: MÜNZER, Jan-Karsten, 25840 Friedrichstadt (DE); RATKE, Andre, 45731 Waltrop (DE)
(86) Internationale Anmeldenummer: PCT/EP2009/001979
(87) Internationale Veröffentlichungsnummer: WO 2009/121480

(56) Entgegenhaltungen:
- WO-A-03/008117
- DE-A1- 3 138 215
- DE-A1- 3 440 315
- DE-A1- 4 332 241
- DE-U1- 20 015 853
- JP-A- 7 291 237
- ARRANZ MIGUEL ANGEL PRIETO: "The use of aqueous ozone for cleaning operations in breweries" 31. Oktober 2007 (2007-10-31), , VALENCIA SPAIN , XP002540011 Gefunden im Internet: URL:http://ec.europa.eu/environment/life/p roject/Projects/index.cfm?fuseaction=home. showFile&rep=article&fil=OZONECIP-Art2-use ofaqueousozoneforcleaningoperationsinbrewe ries.pdf> Seite 5, Absatz 6

## Beschreibung

Die Erfindung bezieht sich auf eine Vorrichtung gemäß dem Oberbegriff des Patentanspuchs 1 und dabei speziell auf einen Pasteur bzw. eine Pasteurisieranlage.

Bekannt sind u.a. Pasteure bzw. Pasteurisieranlagen zur Hitzebehandlung, d.h. zum Pasteurisieren von in Behältern, z.B. in Flaschen abgefüllten Produkten. Bei bekannten Anlagen dieser Art erfolgt die Hitzebehandlung der durch die Pasteurisieranlage auf einem maschinenintemen Transporteur transportierten Behälter durch Besprühen der Behälter in mehreren Behandlungszonen mit einem erhitzten Behandlungamedium, beispielsweise Wasser in der Form, dass durch entsprechende Wahl der Temperatur der Sprühflüssigkeit in den Behandlungszonen zunächst von Umgebungstemperatur ein zunehmendes Erhitzen der Behälter bis auf eine Pasteurisiertemperatur und anschließen ein zunehmendes Abkühlen der Behälter auf Umgebungstemperatur erfolgt. Aufgrund der herrschenden Temperaturen und der ständigen Feuchtigkeit sind derartige Pasteurisieranlagen äußerst anfällig für Verschmutzungen und Verkelmungen aller Art. So muss insbesondere das Verkelmen der Behandlungszonen durch Mikroorgenismen, Insbesondere auch durch Algen, Pilze, Bakterien und/oder Viren ständig verhindert werden.

Hierfür ist es bei bekannten Pasteurisieranlagen üblich, Biozide In hoher Dosierung einzusetzen, und zwar unter Inkaufnahme des Risikos, dass der Verbraucher oder aber auch das Produkt selbst durch Biozide-Reste, die beispielsweise an den pasteurisierten Behältern anhaften, verschmutzt oder sogar geschädigt werden. Weiterhin stellen die verwendeten Biozide einen nicht unerheblichen Kostenfaktor dar.

Es wurde aber auch bereits vorgeschlagen (Arranz Miguel Angel Prieto: "The use of aqueous ozone for cleaning operations in breweries", 31 Oktober 2007) Ozon für Reinigungs- und/oder Sterilisationszwecke bei Tunnelpasteuren zu verwenden. Bekannt ist es weiterhin (DE 31 38 215 A1), in Tauchbädem von Behälterrelnigungsmaschinen Ozon als Reinigungs- und/oder Sterilisationsmedium für die zu behandelnden Behälter zu verwenden.

Aufgabe der Erfindung ist es, eine Vorrichtung aufzuzeigen, bei der die erforderliche Keimfreiheit und Sterilität zuverlässig ohne den Einsatz von Bioziden gewährleistet ist. Zur Lösung dieser Aufgabe ist eine Vorrichtung entsprechend dem Patentanspruch 1 ausgebildet.

Bei der erfindungsgemäßen Vorrichtung, die zur Behandlung von Flaschen oder dergleichen Behältern mit wenigstens einer Behandlungsflüssigkeit dient und bei der durch herrschende Temperaturen und ständige Feuchtigkeit die Gefahr von Verschmutzungen und Verkeimungen besteht, wird die erforderliche Keimfreiheit und Sterilität ohne den Einsatz von Bioziden durch Verwendung von Ozon (O₃) gewährleistet, welches zumindest an den Bereichen der erfindungsgamäßen Vorrichtung, in denen die Gefahr einer Verschmutzung oder Verkeimung besteht, ausgebracht wird, und zwar bevorzugt mit einem flüssigen Behandlungsmedium, so dass das Ozon dann durch Ausgasen aus dem Behandlungsmedium durch seine grundsätzlich umweltfreundliche Oxidation keimtötend wirkt.

Das Behandlungsmedium für die Ozonbehandlung ist die in der Vorrichtung zum Behandeln der Behälter verwendete Behandlungsflüssigkeit.

Die erfindungsgemäße Vorrichtung ist eine Pasteurisieranlage, bei der zumindest diejenigen Behandlungszonen, die unter Berücksichtigung der ständigen Feuchtigkeit und der herrschenden Temperaturen besonders anfällig für Verschmutzungen und Verkeimungen aller Art sind, mit Ozon behandelt werden. Das Ozon wird dabei bevorzugt der Behandlungs- oder Sprühflüssigkeit für die Behandlung der Behälter beigemischt, und zwar in einer Konzentration, die hoch genug ist, um die für die Vermeidung einer Verkeimung notwendige Reinigungs- und Desinfektionsleistung sicherzustellen, gleichzeitig aber auch so gering ist, dass unerwünschte Effekte, wie beispielsweise Korrosion von aus Metall bestehenden Vorrichtungs- oder Maschinenelementen sowie eine gesundheitliche Gefährdung von Personen oder Bedienungspersonal durch Überschreitung maximal zulässiger Ozon-Konzentrationen am Arbeitsplatz zuverlässig vermieden sind.

Da die erfindungsgemäße Vorrichtung mehrere Behandlungszonen aufweist, durch die die Behälter bei ihrer Behandlung hindurchbewegt werden, ist es vorgesehen , die Ozon-Konzentration für die einzelnen Behandlungszonen individuell einzustellen und/oder zu regeln.

Weiterbildungen, Vorteile und Anwendungsmöglichkeiten der Erfindung ergeben sich auch aus der nachfolgenden Beschreibung von Ausführungsbeispielen und aus den Figuren. Dabel sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an Ausführungsbeispielen näher erläutert. Es zeigen:

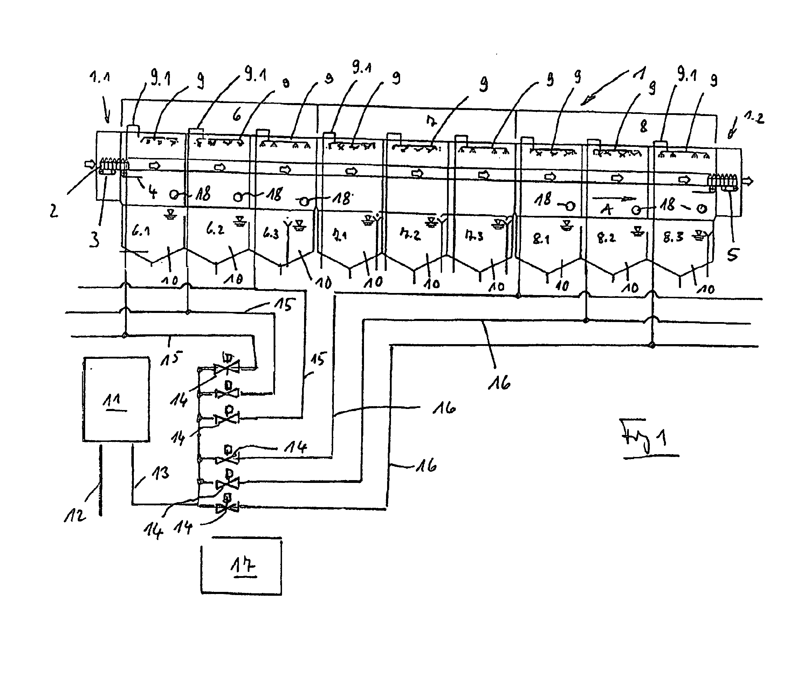

Fig. 1 in vereinfachter schematischer Darstellung eine Maschine zum Behandeln von Flaschen in Form einer Pasteurisieranlage;

In der Figur 1 ist 1 eine Pasteurisieranlage zur Hitzebehandlung, d.h. zum Pasteurisieren eines in Flaschen 2 abgefüllten flüssigen Produktes. Die Flaschen 2 werden nach dem Füllen und Verschließen über einen äußeren Transporteur 3 aufrecht stehend dem Behältereinlass 1.1 der als Tunnelpasteur ausgebildeten Pasteurisieranlage 1 zugeführt und auf einem vorrichtungsinternen Transporteur 4 weiterhin aufrecht stehend durch die verschiedenen Behandlungszonen dieser Anlage an deren Auslass 1.2 bewegt (Pfeil A), von dem die pasteurisierten Flaschen 2 dann über einen äußeren Transporteur 5 einer weiteren Behandlung, beispielsweise einer Etikettleranlage zugeführt werden.

Die Pasteurisieranlage 1 umfasst mehrere Behandlungsabschnitte, und zwar einen Aufheizabschnitt 6, einen Überhitzungs- und Pasteurisierabschnitt 7 und einen Abkühlabschnitt 8, die in dieser Reihenfolge in der Transportrichtung A des Transporteurs 4 aneinander anschließen. Jeder Behandlungsabschnitt ist seinerseits In mehrere In Transportrichtung A aneinander anschließende Behandlungszonen unterteilt, und zwar der Aufheizabschnitt 6 in die Behandlungszone 6.1, 6.2 und 6.3, der Überhitzungsabschnitt 7 In die Behandlungszonen 7.1, 7.2 und 7.3 und der Behandlungsabschnitt 8 in die Behandlungszonen 8.1, 8.2 und 8.3, wobei diese Behandlungszonen wiederum mit steigender Bezugsziffer in Transportrichtung A aneinander anschließen.

Konstruktiv sind die einzelnen Behandlungszonen 6.1 - 6.3, 7.1 - 7.3 und 8.1 - 8.3 insofern identisch aufgebaut, als die Wärmeübertragung auf die Flaschen 2 und damit auf das in diesen enthaltende Produkt oder Füllgut durch Besprühen mit einem flüssigen, Wärme transportierenden Medium, d.h. mit einer Behandlungs- oder Sprühftüssigkeit, beispielsweise Wasser erfolgt, welches jeweils oberhalb des Transporteure 4 aus Sprühanordnungen 9 ausgebracht und unterhalb des Transporteurs 4 in einer Auffangwanne 10 gesammelt wird. Jeder Sprühanordnung 9 Ist eine Versorgungsleitung 9.1 zum Zuführen der Spritzflüssigkeit bzw. des Spritzwassers zugeordnet.

Durch die verschiedenen Behandlungsabschnitte 6, 7 und 8 sowie insbesondere auch durch die Unterteilung dieser Abschnitte in mehrere Behandlungszonen ist ein allmähliches Aufheizen der Flaschen 2 von einer Umgebungstemperatur auf die Überhitzung- bzw. Pasteurisiertemperatur unterhalb 100°C und ein anschließendes allmähliches Abkühlen auf die Umgebungstemperatur möglich.

Speziell In den Behandlungsabschnitten 6.1 - 6.3 und 8.1 - 8.3 besteht wegen der hohen Feuchtigkeit und den herrschenden Temperaturen in hohem Maße die Gefahr einer Verkeimung, wohingegen in den Behandlungsabschnitten 7.1 - 7.3 wegen der wesentlich höheren Temperaturen die Gefahr einer solchen Verkeimung geringer ist. Um eine Verkeimung zu vermeiden und eine eventuell bereits bestehende Verkeimung zu beseitigen, ist vorgesehen, die einzelnen Behandlungszonen 6.1 - 6.3 und 8.1 - 8.3 mit Ozon (O₃) zu beaufschlagen, und zwar mit einer Konzentration oder Dosierung, die für die Vermeidung der Keimbildung ausreicht, aber doch so gering ist, dass unerwünschte Effekte, wie beispielsweise Korrosion von metallischen Maschinen- oder Funktionselementen oder Einbauteilen, aber auch eine Gefährdung von Mitarbeitern und Bedienungspersonal der Pasteurisieranlage durch Überschreitung unzulässig hoher Ozon-Konzentrationen am Arbeitsplatz sicher vermieden sind.

Mit 11 Ist in der Figur 1 eine Ozonanlage bezeichnet, der über eine Leitung 12 Behandlungsflüssigkeit, beispielsweise Wasser aus der Pasteurisieranlage 1 zugeführt wird und in der die Behandlungsflüssigkeit mit Ozon versehen wird, so dass am Auslass der Ozonenlage 11 bzw. an der dortigen Leitung 13 als flüssiges "Konzentrat" die Behandlungsflüssigkeit mit hoher Ozon-Konzentration zur Verfügung steht, d.h. mit einer Ozonkonzentration, die um ein Vielfaches höher ist als die für die eigentliche Ozonbehandlung vorgesehene Konzentration.

Jede Leitung 9.1 ist mit der Leitung 13 über ein eigenständiges, elektrisch steuerberes Dosierventil 14 verbunden, mit dem das Konzentrat aus der Leitung 13 dosiert in die betreffende Leitung 9.1 bzw. in die in dieser Leitung geführte Behandlungsflüssigkeit eingebracht werden kann. Die Dosierventile 14 sind hierfür jeweils in einer zu der Versorgungsleitung 9.1 führenden Verbindungsleitung 15 bzw. 16 vorgesehen.

Zur individuellen Ansteuerung der einzelnen Dosierventile 14 dient eine Steuereinrichtung 17 (Rechner und Analyseeinheit), an die eine Vielzahl von Messstellen oder Messsensoren 18 angeschlossen ist, die jeweils in den einzelnen Behandlungszonen 6.1 - 6.3 bzw. 8.1 - 8.3 die tatsächliche Ozon-Konzentration erfassen und ein dieser Konzentration entsprechendes Messsignal als Ist-Wert an die Steuereinheit 17 liefern, in der dann diese Messsignale parallel oder aber seriell verarbeitet bzw. mit einem für die Behandlungszonen 6.1 - 6.3 bzw. 8.1 - 8.3 jeweils vorgegebenen Soll-Wert verglichen werden, so dass unter entsprechender Ansteuerung der Dosierventile 14 für jede Behandlungszone eine individuelle Regelung zur Erzielung der optimalen Ozonkonzentration möglich ist.

Insbesondere beim Auftreffen der aus der jeweiligen Sprühanordnung 9 ausgebrachten Sprüh- oder Behandlungsflüssigkeit auf Flächen gast das in dieser Flüssigkeit dosiert enthaltene Ozon aus und sorgt für die angestrebte Sauberkeit/Reinheit bzw. für die angestrebte Abtötung von Keimen, Bakterien und Viren Insbesondere an allen Flächen der Behandlungszonen 6.1 - 6.3 bzw. 8.1 - 8.3 und deren Ableitungen sowie auch im gesamten Leitungssystem der Pasteurlsieranlage 1. Zusätzlich gelangt mit der Behandlungsflüssigkeit auch noch nicht ausgegastes Ozon in weitere Behandlungszonen, beispielsweise in die Behandlungszonen 7.1 - 7.3, wo es dann insbesondere auch wegen der dortigen höheren Temperaturen ausgast und ebenfalls seine Wirkung entfaltet.

Durch die Dosierventile 14 besteht insbesondere die Möglichkeit, solche Behandlungszonen 8.1 - 6.3 bzw. 8.1 - 8.3, an denen ein besonders hoher Bedarf an Reinigungs- und/oder Desinfektionsleistung besteht, beispielsweise an den den Behältereinlass 1.1 oder Behälterauslass 1.2 bildenden Behandlungszonen 6.1 bzw. 8.3 sowie an Behandlungszonen mit niedrigerer Temperatur die Ozon-Konzentration höher einzustellen als an anderen Bereichen, in denen ein geringer Bedarf an Reinigungs- und/oder Desinfektionsleistung besteht und an denen somit niedrigere Ozon-Konzentrationen ausreichend sind. Durch die Möglichkeit der individuellen Ozon-Dosierung ist nicht nur für den Behältereinlass 1.1 und den Behälterauslass 1.2 sowie für jede Behandlungszone eine optimale Ozonkonzentration erreichbar, sondern auch ein reduzierter Bedarf und Verbrauch an Ozon, was mit einer erheblichen Kosteneinsparung einhergeht.

Vorstehend wurde davon ausgegangen, dass die dosierte Zugabe von Ozon jeweils an der Zuführleitung 9.1 der Besprühanordnungen 9 folgt. Da bei der Pasteurisieranlage zur Erzielung einer möglichst optimalen Energiebilanz die Behandlungs- oder Sprühflüssigkeit aus den Behandlungszonen des Abkühlabschnittes 8 an Behandlungszonen 6.1 - 6.3 zurückgeführt wird und aus diesen wiederum in Behandlungszonen des Abkühlabschnittes 8 zurück gelangt und außerdem auch ein Austausch der Spritz- und Behandlungsflüssigkeit zwischen den Behandlungszonen 7.1, 7.2 und 7.3 untereinander sowie mit den übrigen Behandlungszonen erfolgt, besteht grundsätzlich die Möglichkeit, das Ozon bzw. die Ozon in hoher Konzentration enthaltende Flüssigkeit auch an anderer Stelle der Pasteurisieranlage 1 zuzuführen, beispielsweise an den Auffangwannen 10 oder an anderen Sammelbahältern der Pasteurisieranlage 1 oder an von den Leitungen 9.1 unterschiedlichen Leitungen usw.

Vorstehend wurde davon ausgegangen, dass die dosierte Zugabe des Ozons unter Verwendung einer mit Ozon versetzten Flüssigkeit, nämlich der Behandlungsflüssigkeit (z.B. Wasser) erfolgt. Grundsätzlich besteht auch die Möglichkeit, das Ozon in Gasform in die Behandlungszonen einzubringen, beispielsweise über Mischdüsen in die Behandlungsflüssigkeit führenden Leitungen oder in die Behandlungsflüssigkeit aufnehmende Behälter usw.

Vorstehend wurde weiterhin davon ausgegangen, dass die dosierte Zugabe von Ozon nur an den Behandlungszonen 8.1 - 6.3 und 8.1 - 8.3 erfolgt. Selbstverständlich besteht die Möglichkeit, die Behandlungszonen 7.1 - 7.3 des Überhitzungsabschnittes 7 ebenfalls mit Ozon zu beaufschlagen, und zwar vorzugsweise wiederum dosiert und bevorzugt unter Verwendung von Messsensoren 18 geregelt.

Die Erfindung wurde voranstehend an Ausführungsbeispielen beschrieben. Es versteht sich, dass zahlreiche Änderungen sowie Abwandlungen möglich sind, ohne dass dadurch der der Erfindung zugrunde liegende Erfindungsgedanke verlassen wird.

**Bezugszeichenliste**

| | |
|---|---|
| 1 | Pasteurisieranlage |
| 2 | Flasche |
| 3 | äußerer Transporteur |
| 4 | innerer Transporteur |
| 5 | äußerer Transporteur |
| 6 | Aufheizabschnitt |
| 6.1 - 6.3 | Behandlungszone des Aufheizabschnittes 6 |
| 7 | Pasteurisierabschnitt |
| 7.1 - 7.3 | Behandlungszone des Posteurisierabschnittes |
| 8 | Abkühlabschnitt |
| 8.1 - 8.3 | Behandlungszone des Abkühlabschnittes 8 |
| 9 | Sprühanordnung |
| 9.1 | Versorgungsleitung |
| 10 | Auffangwanne |
| 11 | Ozonanlage |
| 12, 13 | Leitung |
| 14 | Dosierventil |
| 15, 16 | Verbindungsieltung |
| 17 | Steuereinrichtung (Rechner/Analyseeinheit) |
| 18 | Messsensor |

## Patentansprüche

1. Vorrichtung zum Behandeln von Flaschen oder dergleichen Behältern (2), ausgebildet als Pasteurisieranlage zur Hitzebehandlung bzw. zum Pasteurisieren von in den Behältern (2) abgefüllten Produkten durch Erhitzen, wofür die Behälter (2) In einem Innenraum der Vorrichtung (1) mit einer beispielsweise von Wasser gebildeten erhitzten Behandlungs- oder Sprühflüssigkeit besprüht werden mit wenigstens eine Dosiereinrichtung (14) zum dosierten Einbringen von Ozon in den Innenraum der Vorrichtung (1) **gekennzeichnet durch** mehrere im Innenraum der Vorrichtung (1) gebildete Behandlungszonen (6.1 - 6.3, 7.1 - 7.3, 8.1 - 8.3), in denen die Behälter (2) durch Besprühen mit für die Behandlungszonen (6.1 - 6.3, 7.1 - 7.3, 8.1 - 8.3) unterschiedlich erhitzter Behandlungs- oder Sprühflüssigkeit erhitzt und wieder abgekühlt werden, wobei der Behandlungs- oder Sprühflüssigkeit über die Dosiereinrichtung (14) das Ozon dosiert beigemischt wird und mit der Dosiereinrichtung (14) die Ozonkonzentration in den Behandlungszonen (6.1 - 6.3, 7.1 - 7.3, 8.1 - 8.3; 21, 23, 24, 25), **durch** die die Behälter (2) nacheinander bewegt werden, individuell steuer- oder regelbar ist.

2. Vorrichtung nach Anspruch 1, **dadurch gekennzeichnet, dass** die Dosiereinrichtung (14, 17; 27, 29) für eine dosierte Zugabe eines Ozon in hoher Konzentration enthaltenden flüssigen Konzentrats an die Behandlungsflüssigkeit ausgebildet ist.

3. Vorrichtung nach einem der vorhergehenden Ansprüche, **dadurch gekennzeichnet, dass** die Basis des flüssigen Konzentrats die Behandlungsflüssigkeit der wenigstens einen Behandlungszone (6.1 - 8.3, 7.1 - 7.3, 8.1 - 8.3; 21, 23, 24, 25) ist.

4. Vorrichtung nach einem der vorhergehenden Ansprüche, **dadurch gekennzeichnet, dass** wenigstens ein Dosierventil (14. 27) der Dosiereinrichtung von einer Steuereinrichtung (17, 29) in Abhängigkeit wenigstens eines die Ozonkonzentration erfassenden Messsensors (17, 30) gesteuert wird.

5. Vorrichtung nach einem der vorhergehenden Ansprüche, **dadurch gekennzeichnet, dass** mit der Dosiereinrichtung (14, 17; 27, 29) die Ozonkonzentration in den Behandlungszonen (6.1 - 6.3, 7.1 - 7.3, 8.1 - 8.3; 21, 23, 24, 25), durch die die Behälter (2) nacheinander bewegt werden, individuell steuer- oder regelbar ist.

6. Vorrichtung nach einem der vorhergehenden Ansprüche, **gekennzeichnet durch** ihre Ausbildung als Reinigungsmaschine (20) mit mehreren Behandlungszonen (23, 24) **durch** die die Behälter (2) mit einem maschineninternen Transportsystem (22) bewegt werden, wobei die Dosiereinrichtung (27, 29) zum dosierten Beimischen des Ozons zur Behandlungsflüssigkeit wenigstens einer Behandlungsstation (23, 24) ausgebildet ist.

## Claims

1. Device for treating bottles or similar containers (2), made as a pasteurising installation for the heat-treatment or for the pasteurisation of products filled in the containers (2) by heating, whereby the containers (2) are sprayed in an interior space of the device (1) with a heated treatment or spray liquid in the form for example of water, with at least one dosing device (14) for the dosed introduction of ozone into the interior space of the device (1) **characterised by** a plurality of treatment zones (6.1 - 6.3, 7.1 - 7.3, 8.1 - 8.3) formed in the interior space of the device (1), whereby in said zones the containers (2) are heated and cooled down again by spraying with treatment or spray liquid heated differently for the treatment zones (6.1 - 6.3, 7.1 - 7.3, 8.1 - 8.3), whereby the ozone is added in a dosed manner to the treatment or spray liquid by means of the dosing device (14) and with the dosing device (14) the ozone concentration in the treatment zones (6.1 - 6.3, 7.1 - 7.3, 8.1 - 8.3, 21, 23, 24, 25) through which the containers (2) are moved one after the other, can be individually controlled or adjusted.

2. Device according to claim 1, **characterised in that** the dosing device (14, 17, 27, 29) is designed for a dosed addition of a liquid concentrate containing ozone at a high concentration to the treatment liquid.

3. Device element according to any one of the previous claims, **characterised in that** the basis of the liquid concentrate is the treatment liquid of the at least one treatment zone (6.1 - 6.3, 7.1 - 73, 8.1 - 8.3, 21, 23, 24, 26).

4. Device according to any one of the previous claims, **characterised in that** at least one dosing valve (14, 27) of the dosing device is controlled by a control installation (17, 29) depending on at least one measuring sensor (17, 30) which captures the ozone concentration.

5. Device according to any one of the previous claims, **characterised in that** the ozone concentration in the treatment zones (6.1 - 6.3, 7.1 - 7.3, 8.1 - 8.3, 21, 23, 24, 25), through which the containers (2) are moved one after the other, can be individually controlled or adjusted by the dosing device (14, 17, 27, 29).

6. Device according to any one of the previous claims, **characterised by** their design as a cleaning machine (20) with a plurality of treatment zones (23, 24) through which the containers (2) are moved by a transport system (22) inside the machine, whereby the dosing device (27, 29) is made for the dosed adding of the ozone to the treatment liquid of at least one treatment station (23, 24).

## Revendications

1. Dispositif servant à traiter des bouteilles ou des contenants (2) similaires, se présentant sous la forme d'une installation de pasteurisation aux fins du traitement thermique ou de la pasteurisation de produits transvasés dans des contenants (2) par réchauffage, un liquide de traitement ou un liquide à pulvériser réchauffé formé par exemple d'eau étant pulvérisé sur les contenants (2) dans un espace intérieur du dispositif (1) à l'aide d'au moins un système de dosage (14) servant à introduire de manière dosée de l'ozone dans l'espace intérieur du dispositif (1), **caractérisé par** plusieurs zones de traitement (6.1 - 6.3, 7.1 - 7.3, 8.1 - 8.3) formées dans l'espace intérieur du dispositif (1), dans lesquelles les contenants (2) sont chauffés par la pulvérisation d'un liquide de traitement ou d'un liquide à pulvériser réchauffé de manière diverse pour les zones de traitement (6.1 - 6.3, 7.1 - 7.3, 8.1- 8.3) avant d'être à nouveau refroidis, sachant que l'ozone est mélangé au liquide de traitement ou au liquide à pulvériser de manière dosée par l'intermédiaire du système de dosage (14) et que la concentration d'ozone dans les zones de traitement (6.1 - 6.3, 7.1 - 7.3, 8.1 - 8.3 ; 21, 23, 24, 25), à travers lesquelles les contenants (2) sont déplacés les uns après les autres, peut être commandée ou régulée de manière individuelle à l'aide du système de dosage (14).

2. Dispositif selon la revendication 1, **caractérisé en ce que** le système de dosage (14, 17 ; 27, 29) est réalisé pour une adjonction dosée d'un concentrat liquide contenant de l'ozone en concentration élevée dans le liquide de traitement.

3. Dispositif selon l'une quelconque des revendications précédentes, **caractérisé en ce que** la base du concentrat liquide est le liquide de traitement de la zone de traitement (6.1 - 6.3, 7.1 - 7.3, 8.1 - 8.3 ; 21, 23, 24, 25) au moins au nombre de une.

4. Dispositif selon l'une quelconque des revendications précédentes, **caractérisé en ce qu'**au moins une soupape de dosage (14, 27) du système de dosage est commandée par un système de commande (17, 29) en fonction au moins d'un capteur de mesure (17, 30) détectant la concentration d'ozone.

5. Dispositif selon l'une quelconque des revendications précédentes, **caractérisé en ce que** la concentration d'ozone peut être commandée ou régulée de manière individuelle dans les zones de traitement (6.1 - 6.3, 7.1 - 7.3, 8.1 - 8.3 ; 21, 23, 24, 25), à travers lesquelles les contenants (2) sont déplacés les uns après les autres, à l'aide du système de dosage (14, 17 ; 27, 29).

6. Dispositif selon l'une quelconque des revendications précédentes, **caractérisé par** sa réalisation sous la forme d'une machine de nettoyage (20) comprenant plusieurs zones de traitement (23, 24), à travers lesquelles les contenants (2) sont déplacés à l'aide d'un système de transport (22) propre à la machine, sachant que le système de dosage (27, 29) est réalisé afin de mélanger de manière dosée l'ozone au liquide de traitement au moins d'un poste de traitement (23, 24).
